(19) Europäisches Patentamt

European Patent Office

Office européen des brevets

(11) Veröffentlichungsnummer: **0 455 855 B1**

(12) **EUROPÄISCHE PATENTSCHRIFT**

(45) Veröffentlichungstag der Patentschrift: **12.04.95**

(51) Int. Cl.⁶: **A61C 13/00**

(21) Anmeldenummer: **90108743.7**

(22) Anmeldetag: **09.05.90**

(54) **Verfahren und Vorrichtung zur Erstellung von medizinischen, insbesondere zahnmedizinischen Prothetik- Passkörpern.**

(43) Veröffentlichungstag der Anmeldung:
**13.11.91 Patentblatt 91/46**

(45) Bekanntmachung des Hinweises auf die Patenterteilung:
**12.04.95 Patentblatt 95/15**

(84) Benannte Vertragsstaaten:
**CH DE FR IT LI SE**

(56) Entgegenhaltungen:
EP-A- 0 025 911      EP-A- 0 091 876
EP-A- 0 163 076      DE-A- 2 934 658
DE-A- 3 541 891      US-A- 4 745 290

KONSERVIERENDE ZAHNHEILKUNDE, Heft 3, März 1987, Seiten 1-14; MÖRMANN et al.: "Das Cerec-System: Computergestützte Herstellung direkter Keramikinlays in einer Sitzung"

(73) Patentinhaber: **SIEMENS AKTIENGESELLSCHAFT**
**Wittelsbacherplatz 2**
**D-80333 München (DE)**

(72) Erfinder: **Pfeiffer, Joachim, Dr.-rer.-nat.**
**Jakobsweg, 21**
**D-6140 Bensheim (DE)**

## Beschreibung

Die Erfindung betrifft ein Verfahren zur Erstellung von medizinischen, insbesondere zahnmedizinischen Prothetik-Paßkörpern sowie eine Vorrichtung zur Durchführung des Verfahrens. Unter dem Begriff "Prothetik-Paßkörper" sollen hier sowohl alloplastische also auch endo- und exoprothetische Paßkörper verstanden werden. Im zahnmedizinischen Bereich können diese Inlays, Onlays, Kronen, Brücken, Prothesen oder auch Implantate sein.

Bei der Herstellung solcher Paßkörper wird normalerweise so vorgegangen, daß zunächst von der präparierten Stelle ein Abdruck genommen wird. Anhand des Abdruckes wird im Labor dann ein Arbeitsmodell (Positivmodell) erstellt, von dem schließlich ein Gießmodell (Negativmodell) angefertigt wird. Auf das Arbeitsmodell, welches in der Regel ein Gipsmodell ist, wird Wachs als Formmaterial aufgetragen. Das Wachs wird entsprechend der Form des zu erstellenden Paßkörpers mit einem geeigneten Konturenwerkzeug, z.B. einer Fräse, bearbeitet. Bei diesem Vorgang ist sicherzustellen, daß auf allen Teilen der Umfangsfläche die Wachsschicht eine bestimmte Mindeststärke aufweist.

Eine andere Art der Herstellung von Prothetik-Paßkörpern besteht darin, daß flüssiger Kunststoff in eine dem Prothetik-Paßkörper entsprechende Negativform eingebracht wird und der Kunststoff anschließend, z.B. mittels UV-Strahlen oder Wärme auspolymerisiert wird.

In aller Regel führen die vorgenannten Verfahren zu unerwünschten Formveränderungen beim Erstarren oder Abkühlen.

Die Herstellung der Paßkörper im Gieß- oder Polymerisationsverfahren mit den hierzu notwendigen vorbereitenden Schritten erfordert außerdem einen erheblichen Aufwand, viel Erfahrung und außerordentlich genaue Arbeit.

In der EP-A-0 182 098 und EP-A-0 054 785 werden ein Verfahren sowie eine Vorrichtung zur Herstellung von verwendungsfertigen zahntechnischen Paßkörper beschrieben. Bei diesem Verfahren wird mit Hilfe einer optischen Kamera die Präparationsstelle im Patientenmund abgetastet. Die Meßwerte werden sodann in einen Rechner eingegeben, wo sie zusammen mit weiteren Konstruktionsdaten zu Steuerdaten verarbeitet werden, mit denen schließlich unter Zuhilfenahme einer CNC-Bearbeitungsmaschine der Paßkörper aus einem geeigneten Material ausgearbeitet wird. Obgleich dieses Verfahren sehr vorteilhaft ist, könnte, insbesondere wegen des relativ komplizierten Aufbaus der Kamera, der technische Aufwand noch weiter reduziert werden.

Der im Anspruch 1 angegebenen Erfindung liegt die Aufgabe zugrunde, ein demgegenüber einfacheres und preisgünstigeres Verfahren zur Erstellung eines Prothetik-Paßkörpers sowie eine Vorrichtung zur Durchführung des Verfahrens anzugeben.

Die vorliegende Erfindung geht davon aus, die optische Abtastung nicht vor Ort an der Präparationsstelle vorzunehmen, sondern extern in einer ohnehin vorhandenen Bearbeitungsmaschine, mit deren Hilfe der Paßkörper erstellt wird. Anstelle der bisher notwendigen, relativ aufwendigen optischen Abtastung der Präparationsstelle kann vorteilhafterweise ein relativ einfach vorzunehmender Abdruck treten, der entweder direkt als Negativmodell in die Bearbeitungsmaschine eingesetzt und dort anschließend dreidimensional vermessen wird oder von dem zunächst ein Positivmodell, z.B. ein Gipsmodell, erstellt wird, welches in der Bearbeitungsmaschine vermessen wird. Besonders vorteilhaft ist es, von dem zu erstellenden Paßkörper ein Positiv-Modell anzufertigen und dieses dann in der Bearbeitungsmaschine beitungsmaschine zu vermessen. Aus den gewonnenen Meßdaten werden Steuersignale gebildet, mit denen Antriebs- und Stellmotoren für Bearbeitungswerkzeuge angesteuert werden, die aus einem Werkstückrohling entsprechend den anhand des Modells ausgemessenen und anschließend errechneten Konturen schließlich den Paßkörper fertigen.

Die Erfindung basiert auf der Erkenntnis, die für das Bearbeiten des Paßkörpers ohnehin notwendigen Teile, die die Bearbeitungsmaschine aufweisen muß, insbesondere die exakt bewegbaren und steuerbaren Teile (Freiheitsgrade) der Bearbeitungsmaschine für die Ausmessungen des Modells zu nutzen. Die Aufnahme-und Spanneinrichtung der Bearbeitungsmaschine wird so zweifach genutzt, zunächst, um das Modell zum vorzugsweise optischen Ausmessen, und anschließend, um den Werkstückrohling zur verwendungsfertigen formgebenden Ausarbeitung des Paßkörpers aufzunehmen. Besonders vorteilhaft ist es, das Ausmessen mit Hilfe einer optischen Vermessungseinrichtung durchzuführen, die entweder ortsfest im Gehäuse der Bearbeitungsmaschine oder auf einem verstellbar angeordneten Werkzeugträgerkopf angeordnet sein kann.

Weitere Vorteile ergeben sich aus der nachstehenden Beschreibung eines Ausführungsbeispieles. Es zeigen:

Figur 1 eine Gesamtansicht der erfindungsgemäßen Vorrichtung in schaubildlicher Darstellung,

Figur 2 einen Teil der Vorrichtung nach Figur 1 in einer Schnittdarstellung,

Figur 3 eine schematisierte Darstellung der Steuerung der Antriebs- und Stellmotoren für die Vermessungs- und Bearbeitungsphase,

Figur 4 eine erste Ausführungsform eines Modells,

Figur 5 eine zweite Ausführungsform eines Modells.

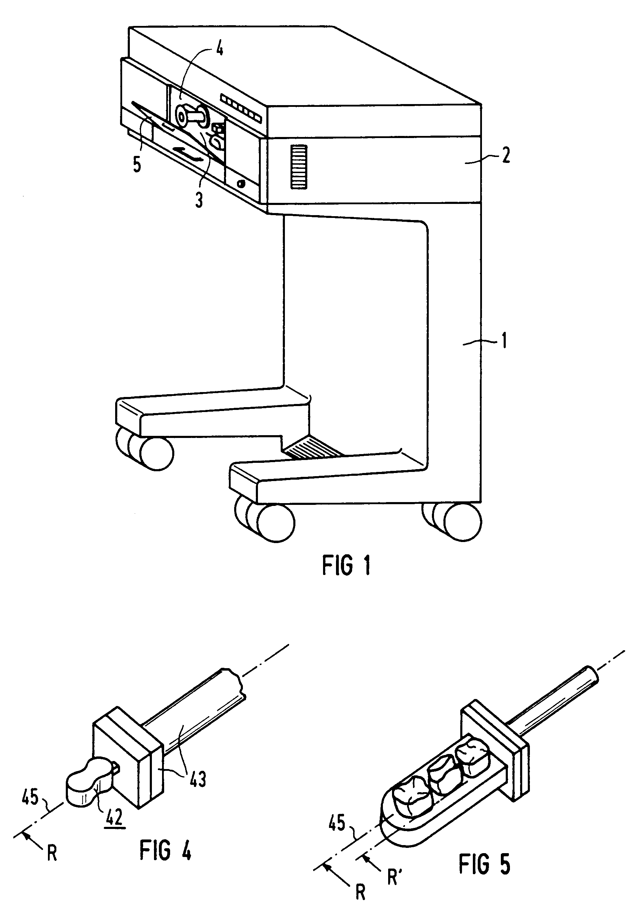

Die Figur 1 zeigt in einer schaubildlichen Darstellung die Gesamtanordnung der erfindungsgemäßen Vorrichtung in einer Ausführungsform zur Herstellung eines Zahnrestaurations-Paßkörpers. Die Vorrichtung enthält ein Fahrstativ 1 mit einem aufgesetzten Gehäuse 2, welches eine allgemein mit 3 bezeichnete Bearbeitungsmaschine enthält. Die einzelnen Komponenten dieser Bearbeitungsmaschine werden anhand der nachfolgenden Figur näher beschrieben. Das Gehäuse 2 bildet eine Bearbeitungskammer 4, die mittels eine vorzugsweise durchsichtigen Deckels 5 bei Betrieb der Maschine geschlossen werden kann.

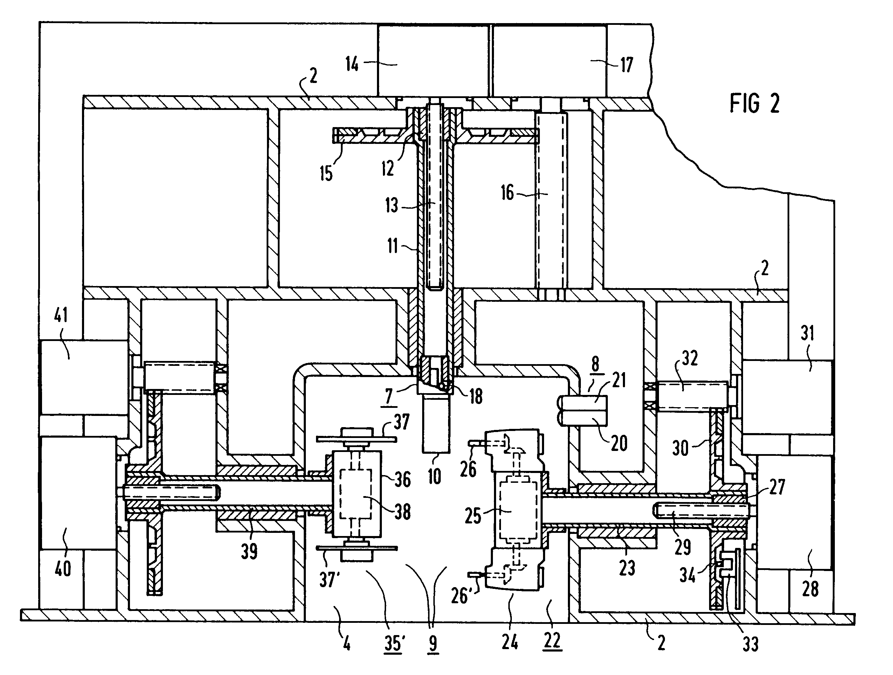

Die Figur 2 zeigt die Bearbeitungsmaschine von oben gesehen im Schnitt. Das Gehäuse 2 nimmt alle drei für die Herstellung des Paßkörpers wichtigen Komponenten auf, nämlich eine Aufnahmeeinrichtung 7, eine Vermessungseinrichtung 8 sowie eine Bearbeitungseinrichtung 9.

Die Aufnahmeeinrichtung 7 dient einerseits dazu, ein Modell von dem Paßkörper oder von dem Objekt, in das der Paßkörper später eingesetzt werden soll, und andererseits (alternativ) einen Werkstückrohling aus einem geeigneten Material, aus dem der Paßkörper erstellt werden soll, aufzunehmen. Das Modell kann ein Positiv- oder Negativ-Modell vom Paßkörper bzw. von dem Objekt sein. In Figur 4 ist beispielsweise ein Positiv-Modell von einem MOD-Inlay-Paßkörper, in Figur 5 ein Positiv-Modell von einem präparierten Zahnobjekt dargestellt.

Die Figur 2 zeigt die Bearbeitungsmaschine in einer zur Bearbeitung eines solchen MOD-Inlays vorbereiteten Phase. Die Aufnehmeeinrichtung 7 ist deshalb bereits mit einem Werkstückrohling 10 , z.B. aus Dentalkeramik, bestückt.

Die Aufnahmeeinrichtung 7 enthält eine als Hohlwelle ausgebildete Spindel 11, die an ihrem dem Werkstückrohling 10 abgewandten Ende eine Spindelmutter 12 aufweist, die mit einer Gewindespindel 13 zusammenwirkt, welche direkt mit der Antriebswelle eines am Gehäuse 2 befestigten Antriebsmotor 14 gekoppelt ist.

Mit der Spindel 11 drehfest verbunden ist ein Zahnrad 15, welches mit einer Zahnwelle 16 in Eingriff steht, die zwischen zwei nicht näher bezeichneten Wandungen des Gehäuses gelagert und ebenfalls direkt mit der Antriebswelle eines zweiten Antriebsmotors 17 gekoppelt ist. Der Antriebsmotor 17 dient dazu, die Spindel 11 in eine Drehbewegung zu versetzen, während mit dem Antrieb 14 eine Linearbewegung (Längsvorschub) der Spindel 11 erzielt wird. Wenn beide Antriebsmotoren 14, 17 gleichzeitig laufen, wird der am vorderen, der Bearbeitungskammer 4 zugewandten Ende der Spindel 11 mittels einer allgemein mit 18 bezeichneten Einspannvorrichtung gehalterte Gegenstand, im vorliegenden Fall der Werkstückrohling 10, spiralförmig bewegt. Es ist verständlich, daß hierzu die Spindel 11 in einem geeigneten Längs- und Drehlager geführt ist.

Die Vermessungseinrichtung 8 besteht im wesentlichen aus einer Lichtquelle 20, die einen scharf gebündelten, auf den in der Aufnahmeeinrichtung 7 gehalterten Gegenstand ausrichtbaren Lichtstrahl erzeugt, und einem optischen Sensor 21, auf dem der von der Lichtquelle 20 auf den Gegenstand projizierte Lichtfleck abgebildet wird.

Die Bearbeitungseinrichtung 9 besteht aus zwei einander gegenüberliegenden Spindelanordnungen 22 und 35, welche bis auf die Anordnung der Werkzeuge völlig gleich ausgebildet sind. Nachfolgend wird deshalb nur der Aufbau der im Bild rechten Spindelanordnung 22 näher erläutert. Die Spindelanordnung 22 enthält eine als Hohlwelle ausgebildete Spindel 23, deren eines Ende in die Bearbeitungskammer 4 ragt und dort einen Werkzeugträgerkopf 24 trägt. Der Werkzeugträgerkopf 24 ist mit der Spindel 23 drehfest verbunden und enthält mittig einen mit 25 angedeuteten Elektromotor, dessen beidseitige Antriebswellen über ein nicht näher bezeichnetes Getriebe zwei gleichartige Bearbeitungswerkzeuge 26, 26' antreiben.

An der Spindel 23 ist eine Spindelmutter 27 befestigt, die mit einer von einem Antriebsmotor 28 angetriebenen Gewindespindel 29 in Eingriff steht. Mit diesen Antriebselementen kann die Spindel 23 in Achsrichtung verstellt werden.

Mit der Spindel 23 drehfest verbunden ist wiederum ein Zahnrad 30, welches mit einer von einem weiteren Antriebsmotor 31 angetriebenen Zahnwelle 32 kämmt. Mit diesem Antriebselement kann der gesamte Werkzeugträgerkopf 24 um die Spindelachse gedreht werden, und zwar um wenigstens 90°, vorzugsweise jedoch um 180°. Ein Drehen um 90° ist insbesondere deshalb angezeigt, um den Werkzeugträgerkopf aus der in der Figur gezeigten, für eine Werkstückbearbeitung geeigneten Arbeitsstellung in eine Grund- oder Ruheposition zu bringen, in der einerseits der Zugang zum Werkstückrohling 10 erleichtert ist und andererseits keine Behinderung beim Ausmessen des Modells, wie später noch erläutert, mit Hilfe der Vermessungeinrichtung 20, 21 gegeben ist.

Nachdem der Vorschub der Spindeln sehr präzise erfolgen muß, ist es notwendig, die hierzu vorgesehen Antriebsmotoren genau anzusteuern. Zu diesem Zweck ist es vorteilhaft, als Antriebsmotoren Schrittmotoren einzusetzen, die an sich in beliebig kleinen Schritteinheiten angesteuert werden können. Um dennoch Schrittverluste ausgleichen zu können, sind die Schrittmotoren mit einer Rückmeldung versehen. Eine solche Rückmeldung kann z.B. darin bestehen, daß in Nähe der Vorschubspindel, oder auch mit dieser gekuppelt, auf Wiederstands-, Kapazitäts-, Induktivitäts-, Ultraschall-, Magnet- und/oder optischer Basis aufbauende Sensormittel vorgesehen sind, die über einen Rechner mit den zugehörigen Schrittmotoren elektrisch verbunden sind. Mit Hilfe eines solchen Wegemeßsystems ist es möglich, die Genauigkeit der Spindelbewegungen zu erhöhen.

Um die Ruhe- oder Ausgangsstellung der Spindeln exakt zu definieren, ist an einer Gehäusewand eine Gabellichtschranke 33 vorgesehen, die mit einem durch einen Schlitz unterbrochenen Ring 34 des Zahnrades 30 derart zusammenwirkt, daß die Spindelstellung exakt definiert ist, und zwar sowohl im bezug auf die Drehstellung als auch in bezug auf die Stellung in axialer Richtung. Während die Position in Richtung der Vorschubachse durch Eintauchen des Ringes 34 in die Gabellichtschranke 33 erfaßt wird, wird die Winkelstellung der Spindel durch ein Signal beim Durchfahren des den Ring 34 unterbrechenden Schlitzes durch die Lichtschranke erfaßt.

Der gegenüberliegend angeordnete Werkzeugträgerkopf 36 ist bezüglich der Verstellung der Spindel gleich aufgebaut; nachdem es sich bei den dort gehalterten Werkzeugen nicht um fingerförmige Bearbeitungswerkzeuge, wie bei den Werkzeugen 26, 26', sondern um scheibenförmige Werkzeuge (Pos 37, 37') handelt, entfällt das beim Werkzeugträgerkopf 24 vorgesehene Winkelgetriebe. Die beiden Antriebswellen des im Werkzeugträgerkopf 36 angeordneten Elektromotors 38 treiben also die beiden Werkzeuge 37, 37' direkt an. Die beiden Antriebsmotoren zum Verstellen der Spindel 39 (analog der Spindel 23) sind mit 40 und 41 bezeichnet.

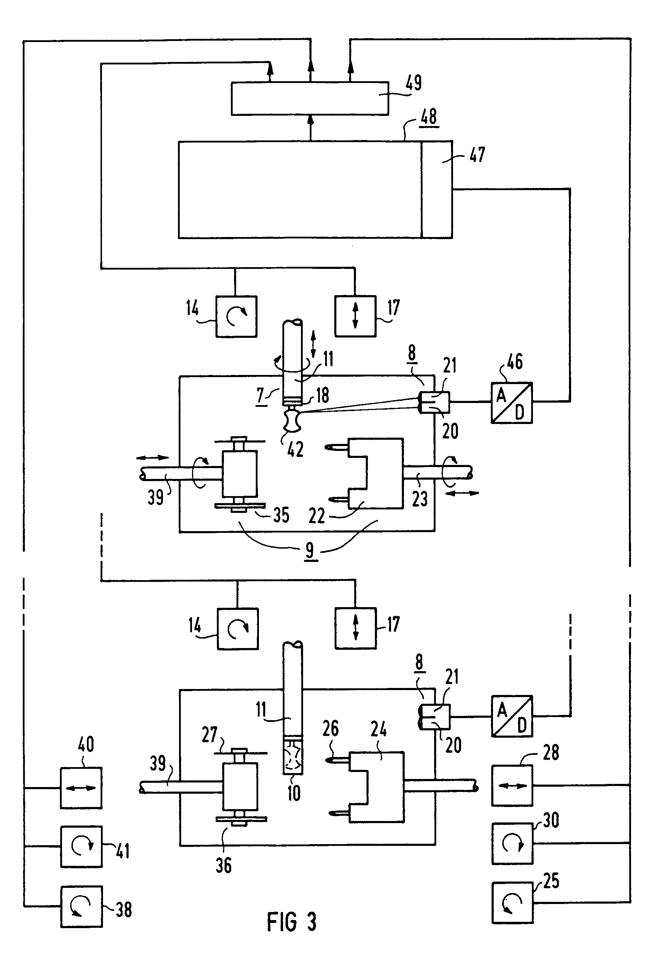

Anhand der Figuren 3 und 4 soll das erfindungsgemäße Verfahren am Beispiel der Erstellung eines MOD-Inlays näher erläutert werden. In Figur 3 der Zeichnung sind die zwei Phasen, einerseits die Vermessungsphase und andererseits die Bearbeitungsphase, sowie die damit zusammenhängende elektrische Verknüpfung der Antriebe schematisch dargestellt.

Im Ausführungsbeispiel wird davon ausgegangen, daß von dem zu erstellenden Paßkörper, also dem MOD-Inlay, zunächst ein Positiv-Modell erstellt wird. Hierzu wird von dem präparierten Zahn, in den der Paßkörper später eingesetzt werden soll, zunächst ein Abdruck und von diesem Abdruck ein Positivmodell vom Inlay erstellt. Dieses Positivmodell, im oberen Teil der Figur 3 mit 42 bezeichnet und in Figur 4 schaubildlich vergrößert dargestellt, kann aus Gips oder Kunststoff erstellt werden und auf einen zapfenförmigen Träger 43 montiert sein. Das Modell 42 wird mit Hilfe des Trägers 43 mit definiertem Anschlag in die mit 18 bezeichnete Schnellspanneinrichtung der Aufnahmeeinrichtung 7 eingesetzt. Danach beginnt die optische Vermessung des Modells. Hierzu sendet die Lichtquelle 20 (FIG 2), die vorzugsweise eine Laserdiode sein kann, einen scharf gebündelten Lichtstrahl aus, der exakt auf die Drehachse 45 des Modells 42 bzw. der Vorschubspindel 11 ausgerichtet ist. Der auf dem Modell projizierte Lichtfleck wird von dem ortsfest angeordneten optischen Sensor 21 erfaßt und in elektrische Signale umgewandelt. Hierzu kann ein geeignetes CCD- oder PSD-Element (position sensitive device) vorgesehen werden. Die Vermessung des Modells erfolgt nach dem Triangulationsverfahren mit einem bestimmten (möglichst kleinen) Parallaxenwinkel zwischen Projektions- und Beobachtungsstrahlengang. Dieses Verfahren ist an sich bekannt und wird beispielsweise bei 3-D-Laserscannern angewandt. Während der Vermessung wird das Modell mit Hilfe der Spindel 11 und der beiden Stellmotoren 14 und 17 spiralförmig mit genügender Feinheit (z.B. mit einer Steigung von 25 $\mu$m) bewegt. In jeder Stellung wird dabei ein Wert $\Delta R = R_2 - R_1$ ermittelt, der der Entfernung der Oberfläche, auf die der Lichtstrahl auftrifft, von der Drehachse des Modells entspricht, d.h., das Modell wird praktisch in Zylinderkoordinaten vermessen.

Die Meßwerte werden in einem A/D-Wandler 46 digitalisiert und anschließend einem Speicher 47 einer Recheneinheit 48 zugeführt. In der Recheneinheit 48 werden die Meßwerte in Vorschubwerte umgerechnet, mit denen dann über ein entsprechendes Interface 49 in der Bearbeitungsphase die Stell- und Antriebsmotoren 14, 17, 25, 28, 31, 38, 40, 41 angesteuert werden.

Nach dem Ausmessen des Modells 42 wird in die Aufnahmeeinrichtung 7 der in Figur 2 mit 10 bezeichnete Werkstückrohling eingesetzt (untere Bildphase in FIG 3). In der anschließenden Bearbeitungsphase wird nun mit Hilfe der beiden Bearbeitungswerkzeuge 26, 37, die in den Werkzeugträgerköpfen 24 und 36 angeordnet sind, der Paßkörper aus dem Werkstückrohling entsprechend dem errechneten Steuerprogramm herausgearbeitet.

Alternativ zu der vorbeschriebenen Verfahrensweise, zunächst von der Präparationsstelle einen Abdruck zu nehmen und davon ein Positiv-Modell vom Paßkörper zu erstellen, ist es denkbar, das Positiv-Modell direkt im Mund mit Hilfe von z.B. Composite-Material zu erstellen. Desgleichen ist es denkbar und im Rahmen der Erfindung möglich, anstelle eines Positiv-Modells vom Paßkörper ein Positv- oder Negativ-Modell von dem Objekt, in das der Paßkörper eingesetzt werden soll, anzufertigen und dieses dann, wie vorbeschrieben, abzutasten. Figur 5 zeigt ein solches Positiv-Modell von einer präparierten Zahnreihe. Bei der optischen Vermessung werden die Außenkonturen des Zahnes sowie die Konturen der mit 44 bezeichneten Kavität erfaßt und aus den Meßwerten dann der Paßkörper errechnet. Diese Variante wäre aber, insbesondere wegen des größeren Rechners, der dazu notwendig wäre, insgesamt technisch aufwendiger, sie hätte jedoch bestimmte Vorteile bei der Erstellung von Gaumenplatten für Vollprothesen, denn hier könnte man den Abdruck sogleich als Negativmodell optisch abtasten und von diesem Negativmodell direkt den Paßkörper, also die Gaumenplatte erstellen. Für die Bearbeitung solcher Prothesenteile ist es vorteilhaft, eine Aufnahmeeinheit vorzusehen, bei der Modell und Werkstück anstelle der beschriebenen kombinierten Dreh- und Vorschubbewegung auf einem Verschiebetisch mit zwei Freiheitsgraden in der Ebene senkrecht zu den Achsen der Werkzeugspindeln verstellt werden.

Um bei gleichem Meßbereich eine bessere Meßgenauigkeit zu erzielen, ist es vorteilhaft, die optische Meßeinrichtung 20, 21 auf einem der Werkzeugträgerköpfe 24, 35 zu montieren. Der Sensor könnte dann über definierte Bewegungen in eine optimale Meßposition gebracht werden. Die Bewegungen, die dann durch die Bewegungen der Werkzeugspindeln erzeugt werden, sind dem Rechner bekannt und können dem eigentlichen Sensormeßwert als Korrektur überlagert werden. Im Extremfall kann der Sensor so gestaltet werden, daß er nicht einen Meßwert über die Entfernung zur Oberfläche des Modells ermittelt, sondern nur signalisiert, ob sich die Oberfläche in einer bestimmten Distanz befindet oder nicht. Dann wird der Sensor bei der Vermessung jeweils so lange verfahren, bis er in dieser bestimmten Distanz ist; der Meßwert der Oberfläche ergibt sich dann aus der Kenntnis des Verfahrweges. Geeignete Detektoren wären zum Beispiel Detektoren, die nach dem Prinzip des konfokalen Mikroskopes arbeiten oder andere Fokus-Detektoren. Abschließend sei darauf hingewiesen, daß die Bewegung des Sensors auch dazu genutzt werden kann, um das Modell aus verschiedenen Blickrichtungen zu vermessen.

**Patentansprüche**

1. Verfahren zur Erstellung von medizinischen, insbesondere zahnmedizinischen Prothetik-Paßkörpern, bei dem von dem Paßkörper oder von dem Objekt, in das der Paßkörper eingesetzt werden soll, zunächst ein Positiv- oder Negativ- Modell (42) erstellt wird, das Modell anschließend in eine Aufnahmeeinrichtung (7) einer Bearbeitungsmaschine (3) eingesetzt und mit Hilfe einer dort angeordneten Vermessungseinrichtung (8) dreidimensional vermessen wird, wobei die Meßdaten einem Rechner (48) zugeführt werden, bei dem nach der Vermessung des Modells (42) in dieselbe Aufnahmeeinrichtung (7) anstelle des Modells (42) ein Werkstückrohling (10) eingesetzt wird, aus dem anschließend der Paßkörper mit Hilfe von mindestens einem Bearbeitungswerkzeug (26, 37) entsprechend den anhand des Modells errechneten Konturen herausgearbeitet wird, wobei mit den aus den Meßdaten gewonnenen Steuersignalen Antriebs- und Stellmotoren (14, 17; 25, 28, 31; 38, 40, 41) für die Aufnahmeeinrichtung (7) und das Bearbeitungswerkzeug (26, 37) zur Bearbeitung des Werkstückrohlings (10) angesteuert werden.

2. Verfahren nach Anspruch 1, bei dem als Modell ein dem Paßkörper entsprechend modellierter Körper verwendet wird.

3. Verfahren nach Anspruch 1, bei dem als Modell ein Abdruck von dem Paßkörper bzw. dem Objekt verwendet wird.

4. Verfahren nach einem der Ansprüche 1 bis 3, bei dem die Konturen des Modells (42) in Zylinder-Koordinaten vermessen werden, indem während der Vermessung das Modell (42) eine Dreh- und gleichzeitig eine Linearbewegung ausführt, und in jeder Stellung des Modells ein Wert, der der Entfernung ( R) eines Punktes der Oberfläche des Modells von deren Drehachse (45) entspricht, ermittelt wird.

5. Vorrichtung zur Durchführung des Verfahrens nach einem der Ansprüche 1 bis 4, enthaltend eine in einem Gehäuse (2) einer Bearbeitungsmaschine (3) angeordnete und in bezug auf eine Ausgangsstellung in wenigstens einem Freiheitsgrad verstellbar ausgebildete Aufnahmevorrichtung (7) zur Halterung einerseits des Modells (42) und - alternativ - andererseits eines Werkstückrohlings (10), eine auf die Aufnahmevorrichtung (7) ausgerichtete Vermessungseinrichtung (8), mit der das Modell dreidimensional vermeßbar ist, enthaltend weiterhin mindestens eine, mindestens ein Bearbeitungswerkzeug (26, 37) aufnehmende Werkzeugspindel (23 39), sowie einen Rechner (48), welcher die aus der Vermessungseinrichtung (8) gewonnene Meßdaten in Steuersignale umwandelt und damit die Antriebe (14, 17; 25, 28, 30; 38, 40, 41) von Werkzeug und/oder Werkzeugspindel sowie der Aufnahmevorrichtung (7) zur Bearbeitung des Werkstückrohlings entsprechend den errechneten Konturen des Paßkörpers steuert.

**6.** Vorrichtung nach Anspruch 5, **dadurch gekennzeichnet,** daß zur Vermessung des Modells (42) eine ortsfeste, im Gehäuse (2) der Bearbeitungsmaschine (3) angeordnete Vermessungseinrichtung (20, 21) vorgesehen ist und die Aufnahmevorrichtung (7) so ausgebildet ist, daß das Modell (42) während der Vermessung gleichzeitig eine Dreh- und eine Linearbewegung ausführt.

**7.** Vorrichtung nach Anspruch 5 oder 6, **dadurch gekennzeichnet,** daß eine optische Vermessungseinrichtung (20/21) mit einer einen scharf gebündelten Lichtstrahl abgebenden Lichtquelle (20) und einem den auf das Modell (42) projizierten Lichtfleck erfassenden optischen Sensor (21) vorgesehen ist.

**8.** Vorrichtung nach Anspruch 7, **dadurch gekennzeichnet,** daß als Lichtquelle eine Laserdiode und als optischer Sensor ein CCD- oder ein PSD-Element (46) vorgesehen sind.

**9.** Vorrichtung nach Anspruch 5, **dadurch gekennzeichnet,** daß die Vermessungseinrichtung (8) an einem das Bearbeitungswerkzeug (26, 37) aufnehmenden und zur Aufnahmeeinrichtung (7) hin und von dieser weg bewegbaren Werkzeugträgerkopf (24, 36) angeordnet ist.

**10.** Vorrichtung nach Anspruch 9, **dadurch gekennzeichnet,** daß die Vermessungseinrichtung aus einem Sensor besteht, der bezüglich der Entfernung zur zu vermessenden Oberfläche lediglich meldet, ob diese in einer vorgegebenen Soll-Entfernung oder abweichend davon näher oder entfernter von dieser liegt.

**11.** Vorrichtung nach Anspruch 10, **dadurch gekennzeichnet,** daß die Vermessungseinrichtung auf einer Fokus-Detektion basiert.

**Claims**

**1.** Method for producing medical, in particular dental prosthetic fitting bodies, wherein in the first instance a positive or negative model (42) is produced of the fitting body or of the object into which the fitting body is to be inserted, the model is subsequently inserted into a receiving device (7) of a processing machine (3) and is measured tridimensionally with the aid of a measuring device (8) that is arranged there, with the measurement data being supplied to a computer (48), wherein after the measurement of the model (42) instead of the model (42) a work piece blank (10) is inserted into the same receiving device (7), from which blank subsequently the fitting body is worked with the aid of at least one processing tool (26, 37) in accordance with the contours calculated with the aid of the model, with drive motors and positioning motors (14, 17; 25, 28, 31; 38, 40, 41) for the receiving device (7) and the processing tool (26, 37) for processing the work piece blank (10) being activated with the control signals obtained from the measurement data.

**2.** Method according to claim 1, wherein a body modelled in accordance with the fitting body is used as a model.

**3.** Method according to claim 1, wherein an impression of the fitting body or of the object is used as the model.

**4.** Method according to one of the claims 1 to 3, wherein the contours of the model (42) are measured in cylindrical coordinates, the model (42) executing a rotary movement and at the same time a linear movement during the measurement, and a value, which corresponds to the distance (R) of a point of the surface of the model from its axis of rotation (45), is determined in each position of the model.

**5.** Arrangement for carrying out the method according to one of the claims 1 to 4, containing a receiving arrangement (7), arranged in a housing (2) of a processing machine (3) and formed so as to be adjustable in relation to a starting position by at least one degree of freedom, for holding, on the one hand, the model (42) and - alteratively - on the other hand, a work piece blank (10); a measuring device (8) which is aligned with the receiving arrangement (7) and with which the model can be measured tridimensionally; containing furthermore at least one tool spindle (23, 39) which receives at least one processing tool (26, 37); and also a computer (48) which converts the measurement data obtained from the measuring device (8) into control signals and thereby controls the drives (14, 17; 25, 28, 30; 38, 40, 41) of tool and/or tool spindle and also of the receiving arrangement (7) for processing the work piece blank in accordance with the calculated contours of the fitting body.

**6.** Arrangement according to claim 5, characterised in that a stationary measuring device (20, 21), which is arranged in the housing (2) of the processing machine (3), is provided for the purpose of measuring the model (42), and the receiving arrangement (7) is formed in such a way that the model (42) simultaneously executes a rotary movement and a linear movement during the measurement.

7. Arrangement according to claim 5 or 6, characterised in that an optical measuring device (20/21) is provided that has a light source (20), which emits a sharply focussed light beam, and an optical sensor (21) which detects the light spot projected onto the model (42).

8. Arrangement according to claim 7, characterised in that a laser diode is provided as a light source and a CCD- or a PSD-element (46) is provided as an optical sensor.

9. Arrangement according to claim 5, characterised in that the measuring device (8) is arranged at a tool carrier head (24, 36) which receives the processing tool (26, 37) and which can be moved towards and away from the receiving device (7).

10. Arrangement according to claim 9, characterised in that the measuring device consists of a sensor which, with regard to the distance to the surface to be measured, merely indicates whether the latter is at a given desired distance or, in departure from this, is closer thereto or more remote therefrom.

11. Arrangement according to claim 10, characterised in that the measuring device is based on focus-detection.

**Revendications**

1. Procédé pour fabriquer des corps ajustés de prothèses médicales, notamment des corps ajustés de prothèses dentaires, selon lequel on établit tout d'abord, à partir du corps ou de l'objet, dans lequel le corps doit être inséré, un modèle positif ou négatif (42), on introduit ensuite le modèle dans un dispositif de logement (7) d'une machine d'usinage (3) et on en effectue une mesure tridimensionnelle à l'aide d'un dispositif de mesure (8) disposé dans cette machine, et selon lequel, après la mesure du modèle (42), on introduit dans le même dispositif de logement (7), à la place du modèle (42), une ébauche (10), à partir de laquelle on forme ensuite, par usinage, le corps ajusté, à l'aide d'au moins un outil d'usinage (26,37) conformément aux contours calculés sur la base du modèle, et selon lequel des moteurs d'entraînement et des servomoteurs (14,17;25,28,31;38,40,41) pour le dispositif de logement (7) et l'outil d'usinage (26,37) sont commandés, pour l'usinage de l'ébauche (10), par les signaux de commande obtenus à partir des données de mesure.

2. Procédé suivant la revendication 1, selon lequel on utilise comme modèle un corps modélisé d'une manière qui correspond au corps ajusté.

3. Procédé suivant la revendication 1, selon lequel on utilise comme modèle une empreinte du corps ajusté ou de l'objet.

4. Procédé suivant l'une des revendications 1 à 3, selon lequel on mesure les contours du modèle (42) en coordonnées cylindriques en exécutant, pendant la mesure du modèle (42), un mouvement de rotation et simultanément un déplacement linéaire, et dans chaque position du modèle, on détermine une valeur qui correspond à l'éloignement ( R) entre un point de la surface du modèle et l'axe de rotation (45) de cette surface.

5. Dispositif pour la mise en oeuvre du procédé suivant l'une des revendications 1 à 4, qui comporte un dispositif de logement (7), qui est disposé dans un carter (2) d'une machine d'usinage (3) et est agencé de manière à être déplaçable suivant au moins un degré de liberté par rapport à une position de départ et qui sert à retenir, d'une part, le modèle (42) et - sinon -, d'autre part, une ébauche (10), un dispositif de mesure (8) orienté sur le dispositif de logement (7) et à l'aide duquel le modèle peut être mesuré dans trois dimensions, et contenant en outre au moins une broche d'outil (23,39), qui loge au moins un outil d'usinage (26,37), ainsi qu'un calculateur (48), qui convertit les données de mesure, obtenues à partir du dispositif de mesure (8), en des signaux de commande et par conséquent commande les dispositifs d'entraînement (14,17; 25,28,30;38,40,41) de l'outil et/ou de la broche d'outil ainsi que le dispositif de logement (7) pour l'usinage de l'ébauche de la pièce, en fonction du contour calculé du corps adapté.

6. Dispositif suivant la revendication 5, caractérisé par le fait que pour la mesure du modèle (42), il est prévu un dispositif fixe de mesure (20,21), qui est disposé dans le carter (2) de la machine d'usinage (3) et que le dispositif de logement (7) est agencé de telle sorte que le modèle (42) exécute simultanément un mouvement de rotation et un déplacement linéaire pendant la mesure.

7. Dispositif suivant la revendication 5 ou 6, caractérisé par le fait qu'il est prévu un dispositif de mesure optique (20/21) comportant une source de lumière (20), qui délivre un faisceau de lumière finement focalisé, et un capteur optique (21), qui détecte le spot de lumière projeté sur le modèle (42).

8. Dispositif suivant la revendication 7, caractérisé par le fait qu'il est prévu comme source de lumière une diode laser et comme capteur optique un élément CCD ou un élément PSD (46).

9. Dispositif suivant la revendication 5, caractérisé par le fait que le dispositif de mesure (8) est disposé sur une tête (24,36) de porte-outil, qui loge l'outil d'usinage (27,37) et est déplaçable en va-et-vient par rapport au dispositif de logement (7).

10. Dispositif suivant la revendication 9, caractérisé par le fait que le dispositif de mesure est constitué par un capteur, qui signale simplement, par rapport à la distance à la surface à mesurer, si cette surface se situe en-deçà d'une distance de consigne prédéterminée ou, contrairement à cela, est plus proche ou plus éloignée de cette distance.

11. Dispositif suivant la revendication 10, caractérisé par le fait que le dispositif de mesure est basé sur une détection du foyer.

FIG 1

FIG 4

FIG 5

FIG 2

FIG 3

11